(19) Europäisches Patentamt
European Patent Office
Office européen des brevets

(11) **EP 3 991 638 A1**

(12) **EUROPEAN PATENT APPLICATION**

(43) Date of publication:
**04.05.2022 Bulletin 2022/18**

(21) Application number: **20204672.8**

(22) Date of filing: **29.10.2020**

(51) International Patent Classification (IPC):
***A61B 5/00*** *(2006.01)*    ***A61B 5/026*** *(2006.01)*
***A61B 5/1455*** *(2006.01)*

(52) Cooperative Patent Classification (CPC):
**A61B 5/0059; A61B 5/0261; A61B 5/7203;**
**A61B 5/14551**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **CSEM Centre Suisse d'Electronique et
de
Microtechnique SA - Recherche et
Développement
2002 Neuchâtel (CH)**

(72) Inventors:
• **Proença, Martin
  1786 Sugiez (CH)**
• **Renevey, Philippe
  1005 Lausanne (CH)**
• **Ferrario, Damien
  1814 La Tour-de-Peilz (CH)**
• **Bonnier, Guillaume
  1030 Bussigny (CH)**

(74) Representative: **P&TS SA (AG, Ltd.)
Avenue J.-J. Rousseau 4
P.O. Box 2848
2001 Neuchâtel (CH)**

(54) **METHOD FOR DETERMINING A PHYSIOLOGICAL PARAMETER USING A PPG SIGNAL WITH REDUCED INFLUENCE OF VENOUS PULSATILITY**

(57)    A method for determining a physiological parameter, comprising: providing a PPG sensor device (1) configured to measure a PPG signal (20); measuring a PPG signal (20) on the user, the PPG signal (20) containing at least two cardiac cycles; identifying PPG pulses (23) from the PPG signal (20), each corresponding to a cardiac cycle; for each PPG pulse (23), determining at least one venous-related feature indicative of the contribution of venous pulsatility to the AC (22); assigning a weighting factor comprising calculating the weighting factor from the set of at least one venous-related feature for each identified PPG pulse; computing a weighted-average PPG pulse by using the PPG pulses (23) and their respective weighting factors; and determining the physiological parameter by using the weighted-average PPG pulse.

| | |
|---|---|
| providing the PPG sensor device | S1 |
| measuring a PPG signal on the user | S2 |
| identifying PPG pulses from the PPG signal | S3 |
| determining at least one venous-related feature per PPG pulse | S4 |
| assigning a weighting factor to each PPG pulse | S5 |
| computing a weighted-average PPG pulse | S6 |
| determining a physiological parameter | S7 |

Fig. 3

**Description**

**Technical domain**

The present invention concerns a method for determining a physiological parameter using a photoplethysmography (PPG) signal with reduced influence of venous pulsatility.

**Related art**

A PPG signal is acquired by illuminating a region of a user's body with a light source and measuring the transmitted light that has gone through the tissue (transmission mode) or that has been back-scattered by the tissue (reflectance mode) with a light receiver. The PPG signal comprises two components: a time non-modulating component (often referred to as direct component, or DC) and a time-modulating component (often referred to as alternating component, or AC). The DC corresponds to the amount of light that the non-pulsating blood and the tissue (muscle, bone, skin, etc.) did not absorb. The AC corresponds to the amount of light that the pulsating blood did not absorb. The AC is thus mainly influenced by arterial pulsatility and by venous pulsatility. Arterial pulsatility corresponds to cardio-synchronous changes in blood volume due to the dilation of the arteries resulting from cardiac activity. Venous pulsatility corresponds to cardio-synchronous changes in blood volume due to the dilation of the veins resulting from cardiac activity.

Consecutive pulses in a PPG signal are not necessarily affected by the same amount of venous pulsatility. For instance, factors such as respiration create an intrathoracic pressure gradient that modulates the venous return to the heart, which in turn modulates differently the amount of venous pulsatility in individual consecutive pulses. Other factors such as the muscle pump system of peripheral veins during activity, an increase in central venous pressure following a decrease in venous compliance, the compression of the vena cava or the effects of gravity, may also affect the amount of venous pulsatility by altering venous return.

Venous pulsatility typically induces blood volume changes of smaller amplitude than arterial pulsatility and is often considered negligible. However, factors such as the pressure applied onto the PPG sensor device against the body affect the amount of venous pulsatility and may let it become significant in some cases, e.g. when not enough pressure is applied. This is particularly true when measuring the PPG signal in reflectance mode. In such cases, separating the pulsatile arterial component from the pulsatile venous component in the measured PPG signal by source separation techniques is extremely complex, as both components originate from the same source (cardiac activity). However, separating both components, or at least obtaining a PPG signal with minimal venous pulsatility influence, is highly desirable for various applications.

Cardiovascular applications which aim at determining a physiological parameter related to the arterial system are generally based on the assumption that the AC of the PPG signal has arterial pulsatility as its only source. Examples of such applications are the estimation of physiological parameters such as blood pressure, blood pressure variations, blood oxygen saturation, perfusion index, stroke volume, stroke volume variations, cardiac output, or cardiac output variations. For instance, the estimation of blood pressure or blood pressure variations is typically based on the analysis of the morphology of the AC of the PPG signal, the latter being assumed to be made of the same physiological determinants as the underlying arterial pressure waveform.

The presence of venous pulsatility changes the morphology of the AC of the PPG signal and therefore adds a confounding factor to its relationship with the underlying arterial pressure waveform, thereby undermining the accuracy of the blood pressure estimation. Similarly, the estimation of blood oxygen saturation or the perfusion index by PPG signal analysis as performed by standard pulse oximeters is intrinsically based on the assumption that the changes in light absorption measured in the AC of the PPG signal are due to changes in oxygen saturation in the arterial blood only. The presence of venous pulsatility modifies the light absorption measured in the AC of the PPG signal and intrinsically invalidates this assumption, affecting the accuracy of the measurement.

The measurement of stroke volume (or any of its related parameters such as stroke volume variations, cardiac output, or cardiac output variations), generally relates an amplitude-related parameter of the AC of the PPG signal to stroke volume, based on the assumption that the amplitude of the PPG signal is related to the distension undergone by the arteries when the stroke volume is ejected into the arterial system. Here again, the presence of venous pulsatility invalidates this assumption as it may modify the amplitude of the AC of the PPG signal.

**Summary**

The present disclosure concerns a method for determining a physiological parameter, comprising the steps of:

> providing a PPG sensor device configured to measure a PPG signal comprising at least an arterial pulsatility component and a venous pulsatility component;

using the PPG sensor device to measure a PPG signal on the user, the PPG signal containing at least two cardiac cycles;

identifying PPG pulses from the PPG signal, each PPG pulse corresponding to a cardiac cycle;

for each identified PPG pulse, determining a set of at least one venous-related feature indicative of the contribution of venous pulsatility to the AC of the identified PPG pulse;

assigning a weighting factor comprising calculating the weighting factor from the set of at least one venous-related feature for each identified PPG pulse;

computing a weighted-average PPG pulse by using the identified PPG pulses and the respective weighting factors; and

determining the physiological parameter by using the weighted-average PPG pulse.

The method disclosed herein allows for determining a physiological parameter with a reduced influence of venous pulsatility.

**Short description of the drawings**

Exemplar embodiments of the invention are disclosed in the description and illustrated by the drawings in which:

Fig. 1 illustrates schematically a reflection-based PPG sensor device comprising a light source and a light receiver;

Fig. 2 illustrates schematically a PPG signal;

Fig. 3 illustrates a method for determining a physiological parameter of a user, according to an embodiment;

Fig. 4 illustrates a method for determining a physiological parameter of a user, according to a specific embodiment;

Fig. 5 illustrates schematically a PPG pulse with amplitude-related, time-related, and area-related features; and

Fig. 6 illustrates schematically PPG spectral power or energy, and various frequency-related features.

**Examples of embodiments**

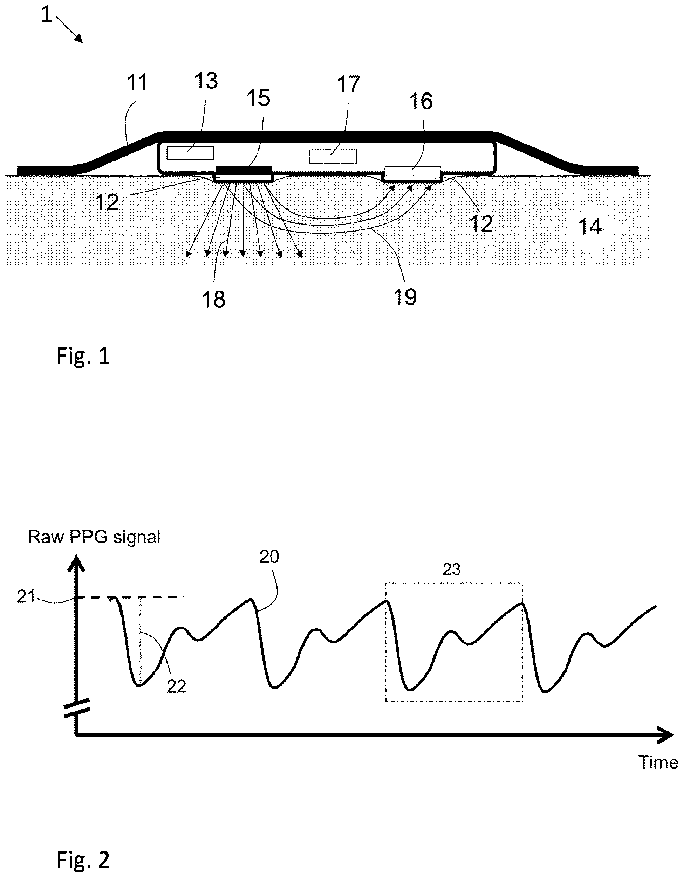

Fig. 1 illustrates schematically a reflection-based PPG sensor device 1 for measuring a PPG signal of a tissue 14 (or body). The PPG sensor device 1 comprises a light source 15, such as a light-emitting diode, and a light receiver 16, such as a photodiode. The light source 15 and the light receiver 16 are on the same side of the tissue 14 to be measured. During a measurement, the light emitted from the light source 15 is either absorbed 18 by the tissue 14 or back-scattered 19 through the tissue 14 to the light receiver 16. The PPG sensor device 1 can further comprise a motion sensor 17 delivering a motion signal representative of a motion of the user. The motion sensor can comprise an accelerometer, a gyroscope, a magnetometer or any suitable sensor configured for measuring a motion signal representative of a body motion of the user. Other configurations of the PPG sensor device 1 can be contemplated. For example, the PPG sensor device 1 can transmission-based PPG sensor.

Fig. 2 shows a PPG signal 20 measured by the PPG sensor device 1. The PPG signal 20 comprises a DC 21 corresponding to the amount of light that the non-pulsating blood and the tissue 14 did not absorb and an AC 22 corresponding to the amount of light that the pulsating blood did not absorb.

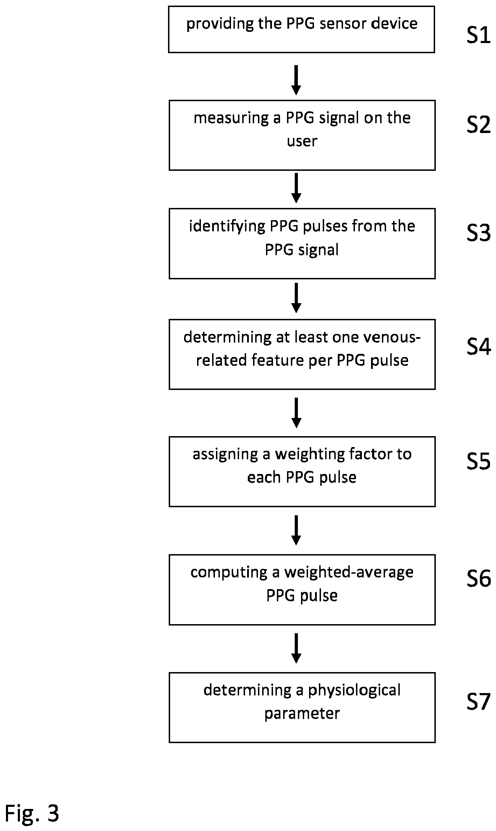

According to an embodiment illustrated in Fig. 3, a method for determining a physiological parameter of a user, comprises the steps of:

providing the PPG sensor device 1 configured to measure the PPG signal 20 comprising at least an arterial pulsatility component and a venous pulsatility component (S1);

using the PPG sensor device 1 to measure a PPG signal 20 on the user, the PPG signal 20 containing at least two cardiac cycles (S2);

identifying PPG pulses 23 from the PPG signal 20, each PPG pulse 23 corresponding to a cardiac cycle (S3);

for each PPG pulse 23, determining at least one venous-related feature indicative of the contribution of venous pulsatility to the AC (S4);

assigning a weighting factor based on the value of the at least one venous-related feature (S5);

computing a weighted-average PPG pulse by using the PPG pulses 23 and their respective weighting factors (S6); and

determining a physiological parameter by using the weighted average PPG pulse (S7).

The PPG sensor device 1 can be provided embedded in or attached to a wearable textile support 11 (see Fig. 1) destined to be worn by the user. The wearable textile support 11 can comprise a patch-like support, a belt, a strap, a garment, or any other suitable wearable textile support.

The PPG sensor device 1 can further be provided as a standalone sensor.

In an embodiment, measuring the PPG signal 20 on the user is performed by placing the PPG sensor device 1 at the thorax or the upper arm, above the level of the seventh intercostal space. By analysing PPG pulses 23 from the PPG signal 20 measured at the upper thorax and the upper arms (above the level of the seventh intercostal space), the present inventors have found that a set of one or more features that are related to the amount of venous pulsatility in each PPG pulse 23 can be calculated. Using said features, it is possible to obtain a PPG pulse 23 where the influence of venous pulsatility is reduced.

Respiration-induced deformation of the thoracic cage may affect the pressure applied onto the sensor, especially when the sensor is placed onto a body part that is particularly sensitive to such deformation. The mechanical modification of the interface between the sensor and the tissues may affect the signal physiologically by modulating the venous blood flow and optically by changing the optical properties of the interface. The method disclosed herein allows for decreasing the influence of venous pulsatility in the measured PPG signal. For example, reliable PPG signals with reduced influence of venous pulsatility can be obtained in the upper thoracic area.

The PPG sensor device 1 can further be provided such that a controlled pressure is applied between the PPG sensor device 1 and the tissue 14 (for example skin), such that substantially the totality of the light emitted by the light source 15 is transmitted to the tissue 14.

In one aspect, the wearable textile support 11 is configured to apply the controlled pressure between the PPG sensor device 1 and the tissue 14. To that end, the wearable textile support 11 can be at least an elastic (stretchy) portion.

In another aspect, the PPG sensor device 1 can be configured to apply the controlled pressure between the PPG sensor device 1 and the tissue 14. For example, the PPG sensor device 1 can comprise a (preferably transparent) protrusion 12 (see Fig. 1) extending between the PPG sensor device 1 and the tissue 14 (or any type of mechanical device) when the PPG sensor device 1 contacts the tissue 14.

In yet another aspect, the controlled pressure can be applied by the user itself, or by gravity.

Because arterial blood pressure is higher than venous blood pressure in the human body, applying a controlled pressure that is above venous pressure but below arterial pressure can decrease the influence of venous pulsatility in the PPG signal 20 and provide a PPG signal that is more representative of arterial pulsatility. The applied controlled pressure can typically be between 0.6 kPa and 10.7 kPa (between about 5 and 80 mmHg). Preferably, the applied controlled pressure can be between 1.3 kPa and 8 kPa (between about 10 and 60 mmHg).

In one aspect, the step of identifying each PPG pulse 23 from the PPG signal 20 can be performed by any one of the following methods: detecting the onset of the identified PPG pulse 23 from the maximum of its first time-derivative; detecting the onset of the identified PPG pulse 23 as the foot 35 of the identified PPG pulse 23; detecting the onset of the identified PPG pulse 23 from the maximum of its second time-derivative; detecting the onset of the identified PPG pulse 23 as the maximum 33 of the identified PPG pulse 23; detecting the onset of the identified PPG pulse 23 as the partial amplitude of the upstroke of the identified PPG pulse 23; detecting the onset of the identified PPG pulse 23 by using the intersecting tangent method (see Reference 1: Chiu, Y. C., et. al., "Determination of pulse wave velocities with computerized algorithms", American Heart Journal, 1991 May;121(5):1460-70).

The step of identifying each PPG pulse 23 from the PPG signal 20 can further be performed by fitting a parametric model such as a hyperbolic tangent or a Morlet wavelet on the pulse upstroke (see Reference 2: Josep Sola, et. al., "Parametric estimation of pulse arrival time: a robust approach to pulse wave velocity", Physiol. Meas. 2009 Jul; 30(7):603-15). The step of identifying each PPG pulse 23 from the PPG signal 20 can further be performed by parametric estimation of its pulse arrival time (see Reference 2).

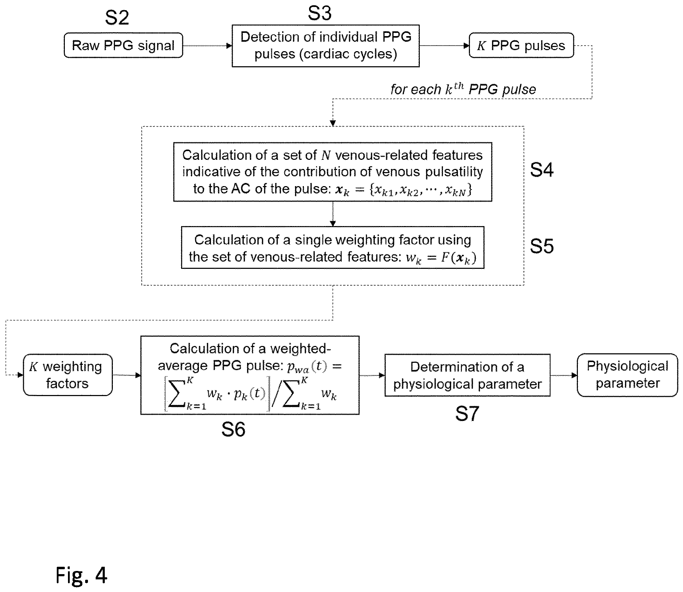

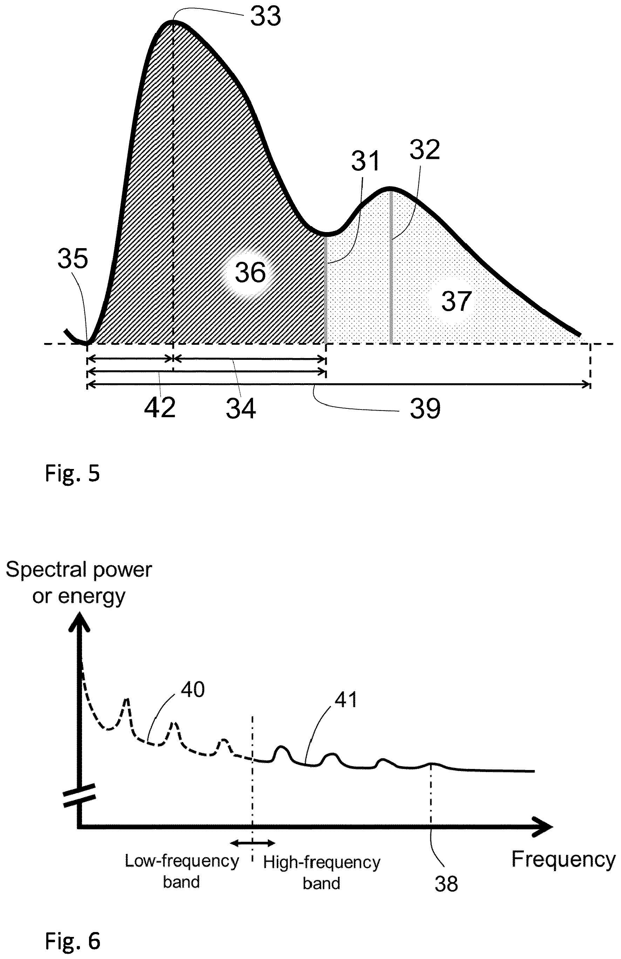

**Fig. 4** illustrates the method for determining a physiological parameter of a user, according to a specific embodiment. In particular, the step of determining at least one venous-related feature (S4) comprises, for each PPG pulse 23, calculating a plurality of venous-related features. In other words, the step of determining at least one venous-related feature (S4) comprises, for each $k^{th}$ PPG pulse 23 ($k \in \{1,..., K\}$), calculating a set $\boldsymbol{x}_k$ of $N$ ($N \geq 1$) venous-related features: $\boldsymbol{x}_k = \{x_{k1}, x_{k2}, \cdots, x_{kN}\}$. The venous-related feature can comprise an amplitude-related feature, a time-related feature, an area-related feature, a frequency-related feature. **Fig. 5** illustrates schematically a PPG pulse 23 showing amplitude-related features 31, 32, 33, time-related features 34, 39, 42 and area-related features 36, 37.

The step of determining at least one venous-related feature (S4) can further comprise calculating at least one transformed venous-related feature by applying a transformation function to at least one venous-related feature. The transformation function can comprise a logarithm, a polynomial regression, or any type of mathematical operation.

The step of determining a least one venous-related feature (S4) can further comprise determining a plurality of venous-related features. At least one combined venous-related feature can then be calculated comprising a combination of any one of the determined venous-related features. For example, determining at least one venous-related feature can comprise calculating a combined venous-related feature $x_{k3}$ from at least two venous-related features, for example: $x_{k3} = x_{k1} \cdot x_{k2}$.

A combined venous-related feature can further be obtained by combining at least two transformed venous-related features. Alternatively, a combined venous-related feature can be obtained by applying the transformation function to the combination of any one of the plurality of venous-related features.

Examples of an amplitude-related feature comprise an absolute amplitude or a relative amplitude determined in the identified PPG pulse 23. Examples of a time-related feature comprise an absolute time interval or a relative time interval determined in the identified PPG pulse 23. Examples of an area-related feature comprise an absolute area or a relative area determined in the identified PPG pulse 23. Examples of a frequency-related feature comprise an absolute frequency, a relative frequency, an absolute spectral power or energy, or a relative spectral power or energy.

Examples of an absolute amplitude comprise the DC 21 or the AC 22 of the PPG pulse 20, or any indicator of the variability of the baseline of the identified PPG pulse 23. For instance, the standard deviation of the baseline of the identified PPG pulse 23. Examples of a relative amplitude comprises the perfusion index (AC/DC), the relative height of the dicrotic notch 31 (see Fig. 5), the relative height of the diastolic peak 32, or any ratio of two absolute amplitudes.

Examples of an absolute time interval comprise the time interval 34 between the systolic peak 33 and the dicrotic notch 31 of the identified PPG pulse 23, or the time interval 42 between the foot 35 and the dicrotic notch 31. Examples of a relative time interval comprise any absolute time interval divided by another time interval, or the duration 39 of the identified PPG pulse 23.

Examples of an absolute area comprise the area under the systolic part 36 or the diastolic part 37 of the PPG pulse, or under the entire PPG pulse 23. Examples of a relative area comprises any absolute area divided by another.

Fig. 6 illustrates schematically PPG spectral power or energy of a PPG pulse 23, and various frequency-related features. An example of an absolute frequency comprises the highest distinguishable cardiac harmonic frequency 38 in the PPG pulse 23. An example of a relative frequency comprises the highest distinguishable cardiac harmonic frequency multiplied by the duration 39 (see Fig. 5) of the PPG pulse 23.

Examples of a spectral power or energy comprise the spectral power or the energy in a low-frequency band 40 or high-frequency band 41 (see Fig 6). Examples of a relative spectral power or energy comprise any absolute spectral power or energy, divided by another or by the total spectral power or energy of the PPG 23.

The step of assigning a weighting factor can comprise using the set of at least one venous-related feature $\boldsymbol{x}_k$ to calculate a weighting factor $w_k$ for each $k^{th}$ identified PPG pulse 23 such that $w_k = F(\boldsymbol{x}_k)$, where $F$ is a weighting function that can comprise any type of mathematical operator that takes as input a set of one or more venous-related feature $\boldsymbol{x}_k$ and outputs a single weighting factor $w_k$.

In one aspect, the weighting function $F$ is a classifier configured such that the value of the weighting factor $w_k$ depends on the value of each of the various venous-related features from the set of at least one venous-related feature $\boldsymbol{x}_k$.

Examples of classifiers comprise artificial neural networks or decision trees.

In another aspect, the weighting factor $w_k$ can be determined by using a linear or non-linear regression of one or more venous-related features $\boldsymbol{x}_k$.

In one aspect, the at least one venous-related feature correlates positively or negatively with the contribution of venous pulsatility to the AC 22 of each PPG pulse 23. In other words, the at least one venous-related feature can have a value that decreases with an increasing contribution of the venous pulsatility to the AC 22 of each PPG pulse 23 or a value that increases with an increasing contribution of venous pulsatility to the AC 22 of each PPG pulse 23.

In particular, each venous-related feature in the set of one or more venous-related features $\boldsymbol{x}_k$ can be configured such that its value correlates positively or negatively with the contribution of venous pulsatility to the AC 22 of each $k^{th}$ identified PPG pulse 23.

In order to give more weight to the identified PPG pulses 23 with low contribution of venous pulsatility to their AC 22, the weighting function F is configured such that an increase in the value of a venous-related feature that correlates positively with the contribution of venous pulsatility to the AC 22 of the $k^{th}$ identified PPG pulse 23 makes the value of the weighting factor $w_k$ decrease, whereas an increase in the value of a venous-related feature that correlates negatively with the contribution of venous pulsatility to the AC 22 of the $k^{th}$ identified PPG pulse 23 makes the value of the weighting factor $w_k$ increase.

The step of computing a weighted-average PPG pulse (S6) can comprise using the weighting factors $w_k$ to calculate a weighted-average PPG pulse. The influence of PPG pulses with high contribution of venous pulsatility to their AC 22 can be minimized as their corresponding weighting factors are low. More specifically, if $p_k(t)$ represents the $k^{th}$ identified PPG pulse 23 in the measured PPG signal 20 and $p_{wa}(t)$ the weighted-average PPG pulse, then $p_{wa}(t)$ can be obtained by using Equation 1:

$$p_{wa}(t) = [\sum_{k=1}^{K} w_k \cdot p_k(t)] / \sum_{k=1}^{K} w_k. \qquad (1)$$

The step of determining a physiological parameter (S7) can comprise using resulting weighted-average PPG pulse $p_{wa}(t)$ for estimating a physiological parameter in an application where the PPG pulse is assumed to be free of venous pulsatility influence.

In an embodiment, the PPG sensor device 1 can comprise a processing module 13 configured to determine a physiological parameter by using the method described herein.

**Reference numeral used in the figures**

| | |
|---|---|
| 1 | PPG sensor device |
| 11 | textile support |
| 12 | protrusion |
| 13 | processing module |
| 14 | tissue |
| 15 | light source |
| 16 | light receiver |
| 17 | motion sensor |
| 18 | absorbed light |
| 19 | back-scattered light |
| 20 | PPG signal |
| 21 | DC |
| 22 | AC |
| 23 | PPG pulse |
| 31 | dicrotic notch |
| 32 | diastolic peak |
| 33 | systolic peak |
| 34 | time interval between systolic peak and dicrotic notch |
| 35 | foot |
| 36 | systolic part |
| 37 | diastolic part |
| 38 | cardiac harmonic frequency |
| 39 | PPG pulse duration |
| 40 | low-frequency band |
| 41 | high-frequency band |
| 42 | time interval between foot and dicrotic notch |
| $F$ | weighting function |
| $p_k(t)$ | $k^{th}$ identified PPG pulse |
| $p_{wa}(t)$ | weighted-average PPG pulse |
| $\mathbf{x}_k$ | set of venous-related features |
| $x_{k1},...,x_{kN}$ | venous-related features 1 to $N$ |
| $w_k$ | weighting factor |

**Claims**

**1.** A method for determining a physiological parameter, comprising the steps of:

providing a PPG sensor device (1) configured to measure a PPG signal (20) comprising at least an arterial pulsatility component and a venous pulsatility component;

using the PPG sensor device (1) to measure a PPG signal (20) on the user, the PPG signal (20) containing at least two cardiac cycles;

identifying PPG pulses (23) from the PPG signal (20), each PPG pulse (23) corresponding to a cardiac cycle;

for each PPG pulse (23), determining a set of at least one venous-related feature indicative of the contribution of venous pulsatility to the AC (22);

assigning a weighting factor comprising calculating the weighting factor from the set of at least one venous-related feature for each identified PPG pulse (23);

computing a weighted-average PPG pulse by using the PPG pulses (23) and their respective weighting factors; and

determining the physiological parameter by using the weighted-average PPG pulse.

2. The method according to claim 1,
wherein the venous-related feature comprises an amplitude-related feature, a time-related feature, an area-related feature or a frequency-related feature.

3. The method according to claim 1 or 2,
wherein determining at least one venous-related feature comprises calculating at least one transformed venous-related feature by applying a transformation function to said at least one venous-related feature.

4. The method according to claim 3,
wherein the transformation function comprises a logarithm, a polynomial regression or any type of mathematical operation.

5. The method according to any one of claims 1 to 4,
wherein determining at least one venous-related feature comprises determining a plurality of venous-related features; and
obtaining at least one combined venous-related feature by combining any one of said plurality of venous-related features.

6. The method according to claim 3 or 4,
wherein determining at least one venous-related feature comprises:

   determining a plurality of venous-related features;
   calculating at least two transformed venous-related features; and
   obtaining at least one combined venous-related feature comprising a combination of said at least two transformed venous-related features.

7. The method according to claim 6,
further comprising applying the transformation function to the combination of any one of said plurality of venous-related features.

8. The method according to any one of claims 1 to 7,
wherein assigning a weighting factor ($w_k$) comprises using the determined at least one venous-related feature to calculate a weighting factor for each PPG pulse (23) by using a weighting function (F).

9. The method according to claim 8,
wherein the weighting function (F) comprises a classifier configured such that the value of the weighting factor ($w_k$) depends on the value of said at least one venous-related feature.

10. The method according to claim 8 or 9,
wherein the weighting factor ($w_k$) is determined by using an artificial neural network, a decision tree, or a linear or non-linear regression of one or more venous-related features.

11. The method according to any one of claims 1 to 11,
wherein each venous-related feature in the set of at least one venous-related feature correlates positively or negatively with the contribution of venous pulsatility to the AC (22) of each PPG pulse (23).

**12.** The method according to claims 8 and 11,
wherein the weighting function ($F$) is configured such that an increase in the value of a venous-related feature that correlates positively with the contribution of venous pulsatility to the AC (22) of the PPG pulse (23) makes the value of the weighting factor ($w_k$) to decrease, whereas an increase in the value of a venous-related feature that correlates negatively with the contribution of venous pulsatility to the AC (22) of the PPG pulse (23) makes the value of the weighting factor ($w_k$) to increase.

**13.** The method according to any one of claims 1 to 12,
wherein the PPG signal is measured at the thorax or the upper arm, above the level of the seventh intercostal space.

**14.** The method according to any one of claims 1 to 13,
wherein said using the PPG sensor device (1) comprises applying a controlled pressure between the PPG sensor device (1) and the tissue (14).

**15.** The method according to claim 14,
wherein said controlled pressure is between 0.6 kPa and 10.7 kPa.

Fig. 1

Fig. 2

providing the PPG sensor device | S1

↓

measuring a PPG signal on the user | S2

↓

identifying PPG pulses from the PPG signal | S3

↓

determining at least one venous-related feature per PPG pulse | S4

↓

assigning a weighting factor to each PPG pulse | S5

↓

computing a weighted-average PPG pulse | S6

↓

determining a physiological parameter | S7

Fig. 3

**S2**

Raw PPG signal

**S3**

Detection of individual PPG pulses (cardiac cycles)

$K$ PPG pulses

*for each $k^{th}$ PPG pulse*

Calculation of a set of $N$ venous-related features indicative of the contribution of venous pulsatility to the AC of the pulse: $x_k = \{x_{k1}, x_{k2}, \cdots, x_{kN}\}$

**S4**

Calculation of a single weighting factor using the set of venous-related features: $w_k = F(x_k)$

**S5**

$K$ weighting factors

Calculation of a weighted-average PPG pulse: $p_{wa}(t) = \left[\sum_{k=1}^{K} w_k \cdot p_k(t)\right] / \sum_{k=1}^{K} w_k$

**S6**

Determination of a physiological parameter

**S7**

Physiological parameter

Fig. 4

Fig. 5

Fig. 6

Europäisches
Patentamt

European
Patent Office

Office européen
des brevets

**EUROPEAN SEARCH REPORT**

Application Number

EP 20 20 4672

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2015/196257 A1 (YOUSEFI RASOUL [US] ET AL) 16 July 2015 (2015-07-16)<br>* paragraphs [0001] - [0014] *<br>* paragraphs [0077] - [0094] *<br>----- | 1-15 | INV.<br>A61B5/00<br>A61B5/026<br><br>ADD.<br>A61B5/1455 |
| X | US 2015/282746 A1 (YOUSEFI RASOUL [US] ET AL) 8 October 2015 (2015-10-08)<br>* paragraphs [0003] - [0011] *<br>* paragraphs [0033] - [0048] *<br>----- | 1-15 | |
| A | US 2006/241506 A1 (MELKER RICHARD J [US] ET AL) 26 October 2006 (2006-10-26)<br>* paragraphs [0032] - [0033] *<br>----- | 1-15 | |
| A | WO 2012/009350 A1 (UNIV YALE [US]; SHELLEY KIRK H [US]; SILVERMAN DAVID G [US]) 19 January 2012 (2012-01-19)<br>* abstract *<br>* page 2, line 24 - page 3, line 19 *<br>----- | 1-15 | |
| A | CN 104 605 863 B (SHENZHEN INST OF ADV TECH CAS) 25 June 2019 (2019-06-25)<br>* abstract *<br>* paragraph [0070]; figure 1 *<br>----- | 1-15 | TECHNICAL FIELDS SEARCHED (IPC)<br><br>A61B |
| A | WO 2007/147069 A2 (ADVANCED BRAIN MONITORING INC [US]; WESTBROOK PHILIP R [US] ET AL.) 21 December 2007 (2007-12-21)<br>* paragraph [0024] *<br>----- | 14,15 | |

The present search report has been drawn up for all claims

| Place of search | Date of completion of the search | Examiner |
|---|---|---|
| The Hague | 1 April 2021 | Loveniers, Kris |

## ANNEX TO THE EUROPEAN SEARCH REPORT
## ON EUROPEAN PATENT APPLICATION NO.

EP 20 20 4672

This annex lists the patent family members relating to the patent documents cited in the above-mentioned European search report.
The members are as contained in the European Patent Office EDP file on
The European Patent Office is in no way liable for these particulars which are merely given for the purpose of information.

01-04-2021

| Patent document cited in search report | | Publication date | Patent family member(s) | | Publication date |
|---|---|---|---|---|---|
| US 2015196257 A1 | | 16-07-2015 | NONE | | |
| US 2015282746 A1 | | 08-10-2015 | NONE | | |
| US 2006241506 A1 | | 26-10-2006 | CA | 2602870 A1 | 02-11-2006 |
| | | | EP | 1883349 A2 | 06-02-2008 |
| | | | JP | 5284082 B2 | 11-09-2013 |
| | | | JP | 2008538936 A | 13-11-2008 |
| | | | US | 2006241506 A1 | 26-10-2006 |
| | | | US | 2008190430 A1 | 14-08-2008 |
| | | | US | 2015101609 A1 | 16-04-2015 |
| | | | US | 2017049391 A1 | 23-02-2017 |
| | | | WO | 2006116469 A2 | 02-11-2006 |
| WO 2012009350 A1 | | 19-01-2012 | EP | 2593000 A1 | 22-05-2013 |
| | | | US | 2013184594 A1 | 18-07-2013 |
| | | | WO | 2012009350 A1 | 19-01-2012 |
| CN 104605863 B | | 25-06-2019 | NONE | | |
| WO 2007147069 A2 | | 21-12-2007 | US | 2008081961 A1 | 03-04-2008 |
| | | | US | 2010145201 A1 | 10-06-2010 |
| | | | WO | 2007147069 A2 | 21-12-2007 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **CHIU, Y. C.** Determination of pulse wave velocities with computerized algorithms. *American Heart Journal,* May 1991, vol. 121 (5), 1460-70

- **JOSEP SOLA.** Parametric estimation of pulse arrival time: a robust approach to pulse wave velocity. *Physiol. Meas.,* July 2009, vol. 30 (7), 603-15